# NEW EUROPEAN PATENT SPECIFICATION

(11) **EP 0 788 378 B2**
(45) Date of publication and mention of the opposition decision: **18.01.2012**
(45) Mention of the grant of the patent: 02.01.2003
(21) Application number: 95934759.2
(22) Date of filing: 27.10.1995
(51) Int. Cl.: A61L 15/28, A61L 15/60

(54) **WOUND DRESSING**
WUNDVERBAND
PANSEMENT

(30) Priority: 27.10.1994 GB 9421653
(43) Date of publication of application: 13.08.1997
(73) Proprietor: Advanced Medical Solutions Limited, Winsford, Cheshire CW7 3PD (GB)
(72) Inventor: QIN, Yimin, Northwich Cheshire CW9 5RQ (GB); GILDING, Keith, Dennis, Winsford Cheshire CW7 4DL (GB)
(74) Representative: Atkinson, Peter Birch
(86) International application number: PCT/GB1995/002535
(87) International publication number: WO 1996/013282

(56) References cited:
- EP-A- 0 227 955
- EP-A- 0 302 536
- EP-A- 0 431 479
- EP-A- 0 477 979
- EP-A1- 0 106 439
- EP-A1- 0 107 915
- EP-A1- 0 599 589
- EP-A1- 0 617 938
- EP-A2- 0 243 069
- WO-A1-90/01954
- WO-A1-90/10424
- WO-A1-92/03172
- WO-A1-93/19710
- FR-A- 2 663 229
- FR-A1- 2 663 229
- GB-A- 2 093 702
- GB-A- 2 221 620
- US-A- 4 341 207
- US-A- 4 572 906
- US-A- 4 977 892
- US-A- 5 010 883
- Neurosurgery, 1984, 15(1):9-13, abstract (Chitosan: a new topical hemostatic agent for diffuse capillary bleeding in brain tissue)
- J. Oral Maxillofac. Surg. 1991, 49(8): 858-63, abstract (Effect of chitosan on lingual hemostasis in rabbits)
- H. BENNETT, F.A.I.C.: 'Concise Chemical and Technical Dictionary', 1974, EDWARD ARNOLD, NEW YORK, ISBN O71312489X page 558
- DIANE KRASNER: 'Chronic Wound Care', 1990, HEALTH MANAGEMENT PUBLICATIONS, INC., KING OF PRUSSIA, PA article TD TURNER: 'The Development of Wound Management Products', pages 31 - 46
- Muzzarelli R. et al, Biomaterials 9(3), p. 247-252 (1988), abstract (Biological activity of chitosan: ultrastructural study)

## Description

The present invention relates to wound dressings.

For the treatment of many types of wounds, particularly medium to highly exuding wounds (e.g. 2nd and 3rd degree burns, decubitus ulcers and leg ulcers) it is necessary to ensure that bulk exudate is removed from the wound and peripheral skin to reduce or eliminate maceration. Prior art dressings have not always proved satisfactory in venting the large amount of exudate present in a wound. As such, the dressing becomes saturated and this results in maceration and excoriation. Additionally, the dressing may require to be changed relatively frequently and this is a labour intensive operation.

According to the present invention a wound dressing comprises in combination
(i) a first wound contact layer of a woven, non-woven or knitted fibrous material
(ii) a second layer of greater hydrophilicity than the first layer, and
(iii) a breathable film having an increased MVTR capability in the presence of liquid water as compared to moisture vapour alone,
wherein layer (i) comprises calcium alginate, zinc alginate, silver alginate, chitosan, pectin, silver N, O-carboxymethyl chitosan, or silver O-carboxymethyl chitosan and wherein layer (ii) is a felt comprised of sodium alginate/calcium alginate, sodium calcium carboxymethyl cellulose, sodium zinc carboxymethyl cellulose, sodium calcium polyacrylate or sodium calcium carrageenin.

Layer (i) is designed to provide a positive action in assisting healing of the wound and may take various forms (as described later) depending on the type of wound to be treated. The provision of layer (ii) (which is of greater hydrophilicity than layer (i) ensures that exudate present in layer (i) may pass into layer (ii) so as to increase the time before layer (i) becomes saturated. Preferably the hydrophilicity of layer (ii) is at least twice, and more preferably 3 to 5 times, that of layer (i).

Layer (i) (i.e. the wound contact layer) will generally be relatively thin (e.g. 50-1000 microns) and may be such as interact positively with the wound to assist healing thereof. Thus, for example, layer (i) may be one which provides for clotting via agglutination of red cells. Alternatively, the layer may be one which is capable of debriding the wound. A further possibility is for the layer to be one which delivers a component to the wound, e.g. an ion, drug, or anti-microbial agent. The materials which may be used for layer (i) are as follows:
(a) calcium alginate which will provide calcium ions for haemostasis;
(b) zinc alginate to deliver zinc ions into the wound to assist healing;
(c) silver alginate to deliver silver ions as powerful anti-microbial agents to infected wounds;
(d) chitosan to provide haemoglutination (i.e. clotting by gelation of red cells leaving the intrinsic and extrinsic clotting cascade intact). Chitosan also appears to have some beneficial effects on contact allergies and anti-microbial activity by stimulating the oxidative attack of white cells. Chitosan has also been reported to assist healing and reduce scarring;
(e) pectin for stimulating autolysis and wound debridement. The pectin may be provided, for example, as pectin/carboxymethyl cellulose/alginate or pectin/alginate;
(f) silver N,O-carboxymethyl chitosan or silver O-carboxymethyl chitosan;

Layer (i) may be provided as a woven, non-woven or knitted material or as a gel. The layer may be in the form of a "rope" for deep cavities or an amorphous gel for sinuses.

Various species may be incorporated in layer (i) for delivery to the wound, e.g.
simple anti microbial agents (e.g. Zn²⁺ and Ag⁺) and metal ions which are enzyme cofactors
enzymes such as collogenase and metallo proteases such as plasmin or plasminogen which can be dosed into layer (i) to be released into the wound during healing to aid fibrinolysis and reduce scar formation
drugs, such as anti-inflammatories etc., for dermatological application.

Layer (i) will also capture proteins and growth factors from the wound, initially by adsorption and as this layer hydrates later in the healing process these proteins and growth factors will be delivered back to the healing wound.

Layer (ii) is preferably also of a woven, non-woven or knitted fibrous material, e.g. a felt.

Layer (ii) will generally have a thickness of 1000 to 5000 microns, preferably 1000 to 2500 microns and may comprise
(a) sodium alginate/calcium alginate felt (e.g. containing 20-60% sodium);
(b) a sodium calcium carboxymethyl cellulose felt;
(c) a sodium zinc carboxymethyl cellulose felt;
(d) a sodium calcium polyacrylate felt; or
(e) a sodium calcium carrageenin felt.

As explained above, layer (ii) is of greater hydrophilicity than layer (i). The requisite hydrophilicity (rate of exudate absorption) for layer (ii) may be obtained by mixing fibres of varying sodium/calcium ratios (for felts (a), (b), (d), and (e)) and by mixing fibres of varying sodium/zinc ratios (for felt (e)). The absolute capacity of the felt for absorbing exudate may be varied by mixing fibres of varying hydrophilicity. For example the absorption capacity of felts made from CMC, polyacrylate or NOCC, all of which are powerfully hydrophilic, may be lowered by the incorporation of alginate fibres. Alternatively, materials of the requisite absorption capability may comprise alginates co-spun with other polymeric materials as disclosed in our copending U.K. Patent Application No. 9419572.

Layers (i) and (ii) may be joined together, e.g. by needle punching, or may be applied separately to the wound.

The dressing comprising layers (i) and (ii) is associated with a breathable film which is of increased MVTR capability in the presence of liquid water as compared to moisture vapour only. MVTR in the presence of liquid water may be measured by ASTM E96BW whereas MVTR in the presence of moisture vapour alone may be measured by ASTM E96B (water method). Preferably the value of the breathability in the presence of liquid water is at least twice and preferably at least three times that in the presence of moisture vapour alone. The value may be up to 30 or 40 times that for moisture vapour alone. Typically the film will be of a material which has an MVTR in the presence of moisture vapour alone (ASTM E96B) of 2,000 to 2,500 g m⁻² 24hr⁻¹ and an MVTR in the presence of liquid water (ASTM E96BW) in the range 6,000 to 30,000 g m⁻² 24hr⁻¹ (e.g. 6,00 to 10,000 g m⁻² 24hr⁻¹). Typically the film will have a thickness of 30-70 microns more preferably 40-60 microns, e.g. about 50 microns.

The film may for example be of polyurethane. Suitable films are available from Innovative Technologies Limited under the designations IT325, IT425 and IT625.

An adhesive will be provided on the film for bonding the latter two the skin around the wound. The adhesive is preferably a hydroactive adhesive most preferably one which, as a continuous layer having a thickness of 20 microns, has an MVTR of 15,000 g m⁻² 24hr⁻¹ using ASTM E96B. Preferably the combination of the adhesive and film is such as to provide an MVTR of 6,000 to 10.000 g m⁻² 24hr⁻¹. An example of a suitable adhesive is a hydroactive adhesive available from Innovative Technologies under the designation ITHA.

The hydroactive adhesive may be provided as a continuous layer on the film. The coating thickness is preferably in the range 15 to 25 microns e.g. about 20 microns.

Alternatively the adhesive may be a pressure sensitive adhesive provided as a cross-pattern to achieve 20-50% area coverage and to achieve similar MVTRs for the combination of adhesive and film of 6,000 to 10,000 g m⁻² 24hr⁻¹.

When the dressing is applied to a wound, the film will generally simply be laid over the combination of layers (i) and (ii).

In use of the dressing comprising such a film, exudate from the wound will initially be absorbed into layer (ii) and will pass therethrough until it comes into contact with the film. The breathability of the film is increased in contact with the liquid present in layer (ii), the increase being dependant on the amount of exudate present in layer (ii) (a greater amount of exudate in layer (ii) producing a greater increase in the breathability of the film). Moisture is therefore able to vent from layer (ii) via the film at a rate which is greater than the MVTR of layer (ii) which is therefore prevented from becoming saturated.

As the wound begins to dry-up during the healing process, the MVTR of the film decreases so that layer (ii) remains moist and does not dry out, thus facilitating healing.

The invention will be illustrated with reference to the following non-limiting Examples.

### Example 1

A non-woven felt made of chitosan fibres and a non-woven felt of a calcium/sodium alginate were needled together to form a two-layer dressing. The chitosan felt provides a wound contacting layer which promotes healing of the wound and also provides antimicrobial properties for the dressing. The calcium/sodium alginate felt has a high absorption capacity.

This combined dressing has the wound healing properties of the chitosan felt and the absorbency of the calcium/sodium alginate felt. By drawing the fluid away from the wound surface, the wound is kept in a relatively dry condition thereby eliminating build up of wound exudate and remove skin maceration.

### Example 2

A non-woven felt of calcium alginate fibres and a non-woven felt of a calcium/sodium alginate were needled together to form a two-layer dressing. The calcium/sodium alginate contained a minimum of 10% of sodium so as to render it more absorbent than the pure calcium alginate felt.

The calcium alginate fibre was a high M fibre which gels more easily than the high G fibre. On application to a wound, the calcium alginate fibre gels to form a moist protective layer whilst excessive fluid is taken up by the calcium/sodium alginate. The wound is therefore kept in a moist healing environment whilst maceration of healthy skin is prevented by the removal of excessive fluid to the calcium/sodium alginate fibre (the upper layer).

## Claims

1. A wound dressing comprises in combination
(i) a first wound contact layer of a woven, non-woven or knitted fibrous material
(ii) a second layer of greater hydrophilicity than the first layer, and
(iii) a breathable film having an increased MVTR capability in the presence of liquid water as compared to moisture vapour alone,
wherein layer (i) comprises calcium alginate, zinc alginate, silver alginate, chitosan, pectin, silver N, O-carboxymethyl chitosan, or silver O-carboxymethyl chitosan and wherein layer (ii) is a felt comprised of sodium alginate/calcium alginate, sodium calcium carboxymethyl cellulose, sodium zinc carboxymethyl cellulose, sodium calcium polyacrylate or sodium calcium carrageenin.

2. A dressing as claimed in claim 1 wherein the hydrophilicity of layer (ii) is at least twice that of layer (i).

3. A dressing as claimed in claim 2 wherein the hydrophilicity of layer (ii) is 3 to 5 times that of layer (i).

4. A dressing as claimed in any one of claims 1 to 3 wherein layer (i) has a thickness of 50 to 1,000 microns.

5. A dressing as claimed in any once of claims 1 to 4 wherein layer (i) delivers a component to the wound.

6. A dressing as claimed in any one of claims 1 to 5 wherein layer (ii) has a thickness of 1,000 to 5,000 microns.

7. A dressing as claimed in my one of claims 1 to 6 wherein the film has an MVTR in the presence of moisture valour alone of 2,000 to 2,500 g m⁻²24hr⁻¹.

8. A dressing as claimed in any once of claims 1 to 7 wherein the film has an MVTR in the presence of liquid water of 6,000 to 30,000 g m⁻²24hr⁻¹.

9. A dressing as claimed in any one of claims 1 to 8 wherein the film has a thickness of 30-70 microns.

10. A dressing as claimed in any one of claims 1 to 9 wherein the film is of a polyurethane.

11. A dressing as claimed in any one of claims 1 to 10 wherein an adhesive is provided on the film for bonding the latter to skin around the wound.

## Patentansprüche

1. Wundverband, der in Kombination folgendes umfaßt:
(i) eine erste Wundkontaktschicht aus einem gewebten, nichtgewebten oder gewirkten Fasermaterial,
(ii) eine zweite Schicht mit einer größeren Hydrophilie als die erste Schicht, und
(iii) eine atmungsaktive Folie mit einem erhöhten Dampfdurchlässigkeitsvermögen beim Vorhandensein von flüssigem Wasser, verglichen mit Wasserdampf allein,
wobei die Schicht (i) Calciumalginat, Zinkalginat, Silberalginat, Chitosan, Pektin, Silber-N,O-Carboxymethyl-Chitosan oder Silber-O-Carboxymethyl-Chitosan umfasst und wobei die Schicht (ii) ein Filz ist, der aus Natriumalginat/Calciumalginat, Natrium-Calcium-Carboxymethylcellulose, Natrium-Zink-Carboxymethylcellulose, Natrium-Calcium-Polyacrylat oder Natrium-Calcium-Carrageenin besteht.

2. Verband nach Anspruch 1, bei dem die Hydrophilie der Schicht (ii) wenigstens zweimal so hoch ist wie die der Schicht (i).

3. Verband nach Anspruch 2, bei dem die Hydrophilie der Schicht (ii) drei- bis fünfmal so hoch ist wie die der Schicht (i).

4. Verband nach einem der Anspräche 1 bis 3, bei dem die Schicht (i) eine Dicke von 50 bis 1000 Mikrometer hat.

5. Verband nach einem der Ansprüche 1 bis 4, bei dem die Schicht (i) eine Komponente an die Wunde abgibt.

6. Verband nach einem der Ansprüche 1 bis 5, bei dem die Schicht (ii) eine Dicke von 1000 bis 5000 Mikrometer hat.

7. Verband nach einem der Ansprüche 1 bis 6, bei dem die Folie eine Dampfdurchlässigkeitszahl beim Vorhandensein von Wasserdampf allein von 2000 bis 2500 gm⁻²24h⁻¹ hat.

8. Verband nach einem der Ansprüche 1 bis 7, bei dem die Folie eine Dampfdurchlässigkeitszahl beim Vorhandensein von flüssigem Wasser von 6000 bis 30000 gm⁻²24h⁻¹ hat.

9. Verband nach einem der Ansprüche 1 bis 8, bei dem die Folie eine Dicke von 30 bis 70 Mikrometer hat.

10. Verband nach einem der Ansprüche 1 bis 9, bei dem die Folie aus einem Polyurethan besteht.

11. Verband nach einem der Ansprüche 1 bis 10, bei dem auf der Folie ein Klebstoff bereitgestellt wird, um dieselbe an der Haut um die Wunde zu befestigen.

## Revendications

1. Pansement pour blessure qui comprend, combinés,
(i) une première couche en contact issue d'un matériau fibreux tissé, non tissé ou tricoté,
(ii) une seconde couche, de plus grande hydrophilie que la première couche, et
(iii) un film respirant ayant une capacité de MVTR (indice de transmission de vapeur) accrue en présence d'eau à l'état liquide par rapport à de la vapeur d'eau seule,
dans lequel la couche (i) comprend de l'alginate de calcium, de l'alginate de zinc, de l'alginate d'argent, du chitosane, de la pectine, de l'argent N,O-carboxyméthyl chitosane ou de l'argent O-carboxyméthyl chitosane et dans lequel la couche (ii) est un feutre composé d'alginate de sodium/alginate de calcium, carboxymethylcellulose sodique calcique, zinc carboxyméthylcellulose sodique, polyacrylate de sodium et de calcium ou carraghénane sodique calcique.

2. Pansement selon la revendication 1, dans lequel l'hydrophilie de la couche (ii) est au moins le double de celle de la couche (i).

3. Pansement selon la revendication 2, dans lequel l'hydrophilie de la couche (ii) est 3 à 5 fois celle de la couche (i).

4. Pansement selon l'une quelconque des revendications 1 à 3, dans lequel la couche (i) a une épaisseur de 50 à 1000 µm.

5. Pansement selon l'une quelconque des revendications 1 à 4, dans lequel la couche (i) délivre un constituant à la blessure.

6. Pansement selon l'une quelconque des revendications 1 à 5, dans lequel la couche (ii) a une épaisseur de 1000 à 5000 µm.

7. Pansement selon l'une quelconque des revendications 1 à 6, dans lequel le film a un MVTR en présence de vapeur d'eau seule de 2000 à 2500 gm⁻²24h⁻¹.

8. Pansement selon l'une quelconque des revendications 1 à 7, dans lequel le film a un MVTR en présence d'eau à l'état liquide de 6000 à 30000 gm⁻²24h⁻¹.

9. Pansement selon l'une quelconque des revendications 1 à 8, dans lequel le film a une épaisseur de 30-70 µm.

10. Pansement selon l'une quelconque des revendications 1 à 9, dans lequel le film est constitué d'un polyuréthane.

11. Pansement selon l'une quelconque des revendications 1 à 10, dans lequel un adhésif est fourni sur le film pour lier ce dernier à la peau autour de la blessure.
